# EUROPEAN PATENT APPLICATION

(11) **EP 1 825 845 A1**
(43) Date of publication of application: **29.08.2007**
(21) Application number: 07250755.1
(22) Date of filing: 22.02.2007
(51) Int. Cl.: A61K 8/97, A61Q 19/08, A61Q 19/00

(54) **Cosmetic herbal compositions**

(30) Priority: 22.02.2006 US 361060
(71) Applicant: Jan Marini Skin Research Inc., San Jose, California 95119 (US)
(72) Inventor: Marini, Jan L., San José, California 95119 (US)
(74) Representative: Brasnett, Adrian Hugh

(57) **Abstract**

Herbal cosmetic skin care compositions formulated to combat conditions associated with oxidative stress are provided. The compositions contain effective amounts of one of several active agents, including an herbal agent derived from one or more of the plant species, as well as cosmetically acceptable carriers. Other agents that may be included are proteins, peptides, anti-inflammatory agents, and/or vasodilators. These cosmetic compositions find use in improving the appearance of aged or damaged skin.

## Description

### BACKGROUND OF THE INVENTION

Currently, tremendous interest and research is being focused on antioxidants and their value in helping to reverse and prevent free radical damage. In mere seconds, one radical can trigger a chain reaction that produces thousands of additional free radicals. The damage caused by free radicals can range from cell damage to ultimate cell death. Cellular breakdown manifests itself superficially in lines, wrinkles, dry skin, loss of elasticity and skin discoloration. The reactivity of various free radicals and the burden they place on the body in defending against the evolution of pathological conditions is known as oxidative stress. Oxidative stress and the likelihood of damage caused by reactive oxygen species (ROS) may increase over time, resulting in the changes in appearance associated with age.

In the U.S. today there are more adults over the age of 40 than ever before in history. The over 40 population will be in the workforce and will live longer than any previous generation. Additionally, given the increased industrialization of the workplace, people today are continuously exposed to greater and greater levels of harmful agents, including those which cause free radical generation. Never the less, because of new advancements in personal care and health, people also have a greater opportunity to maintain a healthy more youthful, vibrant appearance particularly regarding the look and feel of damaged and aging skin.

Accordingly, there is a continued need to develop new products containing antioxidants that can be more effectively delivered so as to increase the aesthetic and protective benefits offered by this class of compounds. Therefore, herein is presented a novel cosmetic antioxidant composition and treatment designed to prolong and promote a more healthy youthful appearance that involves the topical application to the skin of a cosmetic herbal antioxidant preparation that may include lotions, emollients and moisturizers.

### Relevant Literature

U.S. patent application 2005/0226942 A1.

### SUMMARY OF THE INVENTION

The present invention features a novel cosmetic composition for enhancing the appearance and elasticity of the skin. The topical antioxidant compositions contain combinations of herbal ingredients that are formulated to combat and reduce oxidative stress. In some embodiments, the cosmetic formulations of the invention further comprise one or more of: acylated peptides, vasodilators, anti-inflammatories, or analgesics, and may additionally include skin soothing and moisturizing agents. The combination of agents may provide for a synergistic effect on the skin.

The present invention features herbal cosmetic skin care compositions containing effective amounts of one or a combination of several active agents. Such combinations include one or more components (ingredients) from a variety of herbs, including those from the *Bacopa, Camellia, Curcuma, Silybum and Withania genus of plants. Specifically, the* combinations include a mixture of ground powders and extracts from such species of plants as: *Bacopa australis, Bacopa caroliniana, Bacopa crenata, Bacopa madagascariensis, Bacopa monnieri, Bacopa myriophylloides; Camellia assamica, Camellia oleifera, Camellia parvifolia, Camellia sinensis; Curcuma longa and Curcuma zedoaria; Silybum eburneum, Silybum gonzaloi, Silybum marianum; and Withania coagulens, Withania simonii, and Withania somnifera.*

By "one or more components" this includes at least 2 to 5 components from at least two of the *Bacopa, Camellia, Curcuma, Silybum* and *Withania* genus of plants, such as at least 3 to 5 components from at least three of the *Bacopa, Camellia, Curcuma, Silybum* and *Withania* genus of plants, or at least 4 to 5 components from at least four of the *Bacopa, Camellia, Curcuma, Silybum* and *Withania* genus of plants, or five components from the five species of plants. Examples of at least two components include at least a component from both of the *Bacopa and Camellia; the Bacopa and Curcuma; the Bacopa and Silybum; the Bacopa and Withania; the Camellia and Curcuma; the Camellia and Silybum; the Camellia and Withania; the Curcuma and Silybum; the Curcuma and Withania; and the Silybum and Withania genus of* plants. Examples of at least three components include at least a component from the each of *the Bacopa, Camellia and Curcuma; the Bacopa, Camellia and Silybum; the Bacopa, Camellia and Withania; the Bacopa, Curcuma and Silybum; the Bacopa, Curcuma and Withania; the Bacopa, Silybum and Withania; the Camellia, Curcuma and Silybum; the Camellia, Curcuma and Withania; the Camellia, Silybum and Withania; the Curcuma, Silybum and Withania genus* of plants. Examples of at least four components include a mixture of at least a component from each of the *Bacopa, Camellia, Curcuma* and *Silybum;* the *Bacopa, Camellia, Curcuma* and *Withania;* the *Bacopa, Camellia, Silybum* and *Withania;* the *Bacopa, Curcuma Silybum* and *Withania;* and the *Camellia, Curcuma, Silybum* and *Withania* genus of plants.

The herbal components of the skin care compositions of the present invention are combined with cosmetically acceptable vehicle(s) so as to produce formulations that can be applied topically to decrease oxidative stress, reduce skin irritation and inflammation, as well as to promote a more healthy youthful appearance.

### DETAILED DESCRIPTION OF THE EMBODIMENTS

Topical skin care compositions are herein provided for the treatment of damage associated with disruptions caused by harmful exposure to environmental factors and other inflammatory aggravators that may lead to oxidative stress. The skin care compositions of the invention include combinations of effective amounts of various herbal ingredients that are formulated to treat a variety of aging-related conditions or disorders of the skin and thereby improve the appearance of the skin. Thus in one aspect the invention is a cosmetic, non-therapeutic, treatment of the skin using a composition of the invention. An effective amount of a topical composition of the invention is useful in the treatment of various skin conditions or disorders, such as e.g., dermatitis, eczema, dry skin, acne, sunburn, inflammation, pruritic lesions and other inflammatory and non-inflammatory lesions of the skin of any subject and for improving the appearance of the skin. Insofar as any such uses are non-cosmetic, the invention provides a composition for use in a method of treatment of the skin, e.g. by topical application of the composition.

By an "effective amount" (e.g., of an antioxidant composition) is meant a quantity of the composition provided for topical administration and at a particular dosing regimen, that is sufficient to achieve a desired appearance, therapeutic and/or prophylactic effect. For example, an effective amount may result in the prevention of or a decrease in the symptoms associated with a condition that is being treated. The amount of the herbal composition topically administered to the subject will depend on the type and severity of the condition, the characteristics of the subject; where such characteristics include general health, age, sex, body weight and tolerance to the active agents in the compositions. The skilled practitioner will be able to determine appropriate dosages depending on these and other factors. The compositions of the present invention can also be administered in combination with one or more additional cosmetic, therapeutic or prophylactic compounds for the improvement of the appearance of the skin. The term "subject," as used herein, is preferably a mammal, such as a human.

In one embodiment of the invention, herbal compositions are provided to be topically applied to the skin. Another aspect of the invention is to provide herbal compositions that are formulated for topical administration and applied to the skin so as to reduce oxidative stress. In a further embodiment, the topical herbal compositions are applied so as to give the skin a more vibrant, youthful appearance and to decrease the indicia of aging, e.g., the appearance of wrinkles, inflammation, abrasions and lesions.

These and other aspects of the present invention will be more readily apparent and understood by considering the following description.

### Components of the Cosmetic Compositions

The compositions contemplated for use in the present invention include a mixture of various plant derived ingredients and/or active agents that have been formulated in conjunction with a cosmetically acceptable vehicle so as to be applied topically to the skin to reduce oxidative stress and improve the appearance of the skin. The formulation will comprise at least one, at least two, at least three, at least four, or at least five different plant ingredients described herein, and will further comprise a cosmetically acceptable vehicle. The formulation may further comprise one or more of acylated peptides, proteins as described herein, vasodilators, anti-inflammatories, or analgesics. Such agents may provide for a synergistic skin care composition. As used herein, "different plant ingredients" refer to ingredients derived different plant genera.

The plant ingredients useful in compositions of the invention for the improvement of the appearance of the skin are set forth and described herein below.

*Bacopa is a member of the Plantaginaceae family. Of particular interest is the species Bacopa monnieri. Active agents of* Bacopa monnieri include hersaponin, and bacosides A & B, which may be extracted from the leaves of the plant by methods well known in the art and standardized to provide a minimum level of bacosides A & B from about 10%, 20%, 30% 40%, 50%, 60% etc. which may then be formulated by itself or in conjunction with other herbal ingredients of the invention to from a topical composition. Other species of interest include: *Bacopa australis, Bacopa caroliniana, Bacopa crenata, Bacopa madagascariensis, Bacopa monnieri, and Bacopa myriophylloides. Bacopa extracts are commercially available, or may* be prepared using methods known to those of skill in the art. Bacopa extracts may be present in the compositions of the invention at a concentration of at least about 0.1 %, more usually at least about 0.5%, at least about 1 %, at least about 2.5%, at least about 5%, and not more than about 20% or about 30%, but usually not more than about 10%, or not more than about 15%. The percentage of bacosides in such an extract are typically about 20% to about 60%, more typically about 40% to about 50%, or about 45% bacosides.

*Camellia* is a member of the Theaceae family. Camellia contain a number of active agents, including polyphenols, caffeine, tannins, carotene, catechin, epigallocatechin, epigallocatechin gallate, riboflavin, kaempferol, quercitrin, theophylline, theobromine, xanthine, hypoxanthine, adenine, dextrin, inositol, nicotinic acid, pantothenic acid, malic acid, oxatic acid, and ascorbic acid. A species of interest is *Camellia sinensis.* By using well known means in the art a standardized 50%, 60%, 70%, 80%, 90%, 98%, etc. polyphenol composition may be extracted from the leaves of *Camellia sinensis* Other species of interest *include: Camellia assamica, Camellia oleifera, and Camellia parvifolia. Camellia extracts are* commercially available, or may be prepared using methods known to those of skill in the art. Camellia extracts, for instance Camellia sinensis (i.e., green tea) extracts, may be present in the compositions of the invention at a concentration of at least about 0.1%, more usually at least about 0.5%, at least about 1%, at least about 2.5%, at least about 5%, and not more than about 10% or about 20%, but usually not more than about 7.5%, or not more than about 5%. The percentage of polyphenols in such an extract are typically about 30% to about 99%, more typically about 60% to about 98%, or about 80 % to about 95% polyphenols. Epigallocatechin may be present in the phenol extract at an amount of about 30% to about 60%, about 40% to about 50%, or about 45% EGCG.

*Curcuma* is a member of the *Zingiberaceae* family. Active ingredients include curcumin and tetrahydrocurcuminoid. A species of interest is Curcuma longa. By using well known means in the art a standardized 50%, 60%, 70%, 80%, 90%, 95%, *etc.* Another species of interest is *Curcuma zedoaria. Curcuma* extracts are commercially available, or may be prepared using methods known to those of skill in the art. *Curcuma* extracts, for instance, tumeric, may be present in the compositions of the invention at a concentration of at least about 0.1 %, more usually at least about 0.5%, at least about 1%, at least about 2.5%, at least about 5%, and not more than about 10% or about 20%, but usually not more than about 7.5%, or not more than about 5%. The percentage of curcumin in such an extract are typically about 40% to about 99%, more typically about 60% to about 95%, or about 70% to about 80% curcumin.

*Silybum* is a flowering plant of the Asteraceae family. Active ingredients include Silymarin, which is composed of 4 isomers: silybinin, silychristin, silydianin and isosilybinin. By using well known means in the art a standardized 50%, 60%, 70%, 80%, 90%, 95%, etc. *Silybum* extract may be obtained from the seed of *Silybum marianum, Silybum eburneum,* or *Silybum gonzaloi. Silybum* extracts are commercially available, or may be prepared using methods known to those of skill in the art. *Silybum* extracts, for instance Milk thistle, may be present in the compositions of the invention at a concentration of at least about 1%, more usually at least about 5%, at least about 10%, at least about 15%, at least about 20%, and not more than about 40% or about 30%, but usually not more than about 7.5%, or not more than about 6%. The percentage of Silymarin in such an extract are typically about 50% to about 95%, more typically about 60% to about 90%, or about 70% to about 80% Silymarin.

*Withania* is member of the *Solanaceae.* By using well known means in the art a fine powder may be obtained from *Withania somnifera,* which may then be formulated by itself or in conjunction with other herbal ingredients of the invention to from a topical composition. Other species of interest are *Withania coagulens, and Withania simonii. Withania* extracts are commercially available, or may be prepared using methods known to those of skill in the art. *Withania* extracts for instance, Ashwagandha powder, may be present in the compositions of the invention at a concentration of at least about 1 %, more usually at least about 5%, at least about 7.5%, at least about 10%, at least about 15%, and not more than about 30% or about 20%, but usually not more than about 6%, or not more than about 4%.

An herbal composition of the invention includes as an active agent a component as described above that is derived from at least one plant variety selected from the *Bacopa, Camellia, Curcuma, Silybum,* and/or *Withania* genus. In one embodiment, the herb containing composition of the invention contains at least two active agents derived from at least two plant varieties selected from the *Bacopa, Camellia, Curcuma, Silybum,* and/or *Withania* genus. In one embodiment, the herb containing composition of the invention contains at least three active agents derived from at least three plant varieties selected from the *Bacopa, Camellia, Curcuma, Silybum,* and/or *Withania* genus. In one embodiment, the herb containing composition of the invention contains at least four active agents derived from at least four plant varieties selected from the *Bacopa, Camellia, Curcuma, Silybum,* and/or *Withania* genus. In one embodiment, the herb containing composition of the invention contains at least five active agents one derived from each of the plant varieties: *Bacopa, Camellia, Curcuma, Silybum,* and *Withania* genus.

In one embodiment, a composition of the invention includes at least one extract or active agent derived from a component from at least one of *Bacopa monniera, Camellia sinensis, Curcuma longa, Silybum marianum, and*/*or Withania somnifera species. In one embodiment,* a composition of the invention includes at least two extracts or active agents derived from a component from at least two of Bacopa monniera, Camellia sinensis, Curcuma longa, Silybum marianum, and/or Withania somnifera species. In one embodiment, a composition of the invention includes at least three extracts or active agents derived from a component from at least three of Bacopa monniera, Camellia sinensis, Curcuma longa, Silybum marianum, and/or Withania somnifera species. In one embodiment, a composition of the invention includes at least four extracts or active agents derived from a component from at least four of Bacopa monniera, Camellia sinensis, Curcuma longa, Silybum marianum, and/or Withania somnifera species. In one embodiment, a composition of the invention includes at least five extracts or active agents derived from a component from at least each of Bacopa monniera, Camellia sinensis, Curcuma longa, Silybum marianum, and/or Withania somnifera species. For instance, in one embodiment a composition of the invention includes from 1 to 20% by weight Bacopa monnieri; from 1 to 20% by weight Camellia sinensis; from 1 to 20% by weight Cucuma longa; from 1 to 20% by weight Silybum marianum; from 1 to 20% by weight Withania somnifera; and a cosmetically acceptable vehicle.

In one embodiment, the active agent is a finely ground powder or an extract derived from a component of one or more of the above listed plant varieties. In one embodiment, a composition of the invention includes as an active agent one or more of bacoside A, bacoside B, polyphenol, curcumin, silymarin, and an ashwagandha powder. Accordingly, a herb-containing composition of the invention can include as active agents components from any or all of the plant varieties set forth above in any quantity, or combination, suitable to give the desired skin rejuvenating effect when applied topically.

The cosmetic compositions of the present invention are formulated for topical use. A component from the one or more plant varieties detailed herein may serve as an active agent and may be incorporated into a topical composition by itself or in combination with one or more of the other plant derived active agents. In general, the subject cosmetic compositions contain at least about 1%, at least about 2.5%, at least about 5%, and not more than about 50% (weight/weight) of herbal components, usually not more than about 20% or about 25%(weight/weight) of herbal components.

The cosmetic compositions of the present invention find use in the treatment of conditions and disorders associated with the skin, including but not limited to: dermatitis, eczema, dry skin, acne, sunburn, inflammation, pruritic lesions and other inflammatory and non-inflammatory lesions of the skin. Accordingly, the cosmetic compositions of the present invention, including various combinations of the above set forth herbal components, are useful for improving the appearance of the skin.

In many embodiments, additional ingredients are included as part of the subject cosmetic compositions to enhance the synergistic effect of the active agents of the herbal compositions. Accordingly, one or more of the following ingredients may be included in a cosmetic formulation of the present invention for the improvement of the appearance of the skin.

### Peptides

Peptides, including but not limited to: di-, tri-, tetra-, and pentapeptides, as well as oligo-peptides of from about 6 to about 30 amino acids in length and derivatives thereof, may be included as cosmetic benefit agents of the present invention in amounts that are safe and effective. In some embodiments, such peptides are usually acylated, and comprise at least one lipid moiety, which moiety may be myristoyl, palmitoyl, etc., which increases the hydrophobicity of the peptide. Myristoyl pentapeptides are of particular interest, as is the use of thymosin β4. As used herein, "peptides" refers to both naturally occurring peptides and synthesized peptides. Below is a non-limiting list of exemplary peptide agents that find use in the cosmetic compositions of the present invention.

Certain peptide agents of interest stimulate the macromolecules of the dermis. For example, synthetic peptides such as lamin, biopeptide CL or palmitoyloligopeptide (SEDERMA) activate the synthesis of collagen. In addition, natural peptides extracted from plants, such as the soya bean hydrolyzate marketed by the company COLETICA under the trademark Phytokine™, also provide this activity.

Certain other peptide agents act on the synthesis of fibronectin, such as the palmitoyl pentapeptide marketed by the company SEDERMA under the trademark Matrixil™. Still other of these peptides inhibit metalloproteinases, such as oligopeptides and lipopeptides, lipoamino acids, and malt extract marketed by the company COLETICA under the trademark Collalift™, while some peptide agents inhibit serine proteases, such as leukocyte elastase or cathepsin G, including the peptide extract of seeds of leguminous plants *(Pisum sativum)* which is marketed by the company LSN under the trademark Parelastyl™ and certain pseudo-dipeptides.

Some peptide agents that find use in the cosmetic compositions of the present invention stimulate the proliferation of fibroblasts, including plant polypeptides (or extracts) from soya bean (e.g., Eleseryl SH-VEG 8™ marketed by the company LSN, or Raffermine™ marketed by the company SILAB).

In addition to the activities noted above, certain peptide agents may provide antibacterial or anti-fungal activity to the cosmetic compositions of the invention. For example, see US patent 6,835,536, which describes numerous cationic antimicrobial peptides (e.g. indolicidins or analogs or derivatives thereof derived from natural sources or produced synthetically) that find use in topically-applied compositions for the treatment of acne. Similarly, U.S. Patent no. 6,713,078 describes granulysin peptides useful in the topical treatment of acne.

The peptide agents of the present invention are formulated at an effective concentration within the subject cosmetic compositions, meaning at a concentration that provides the intended benefit when applied topically. An effective concentration of peptide or peptide-like compounds is preferably in a range of at least about 0.5%, more usually at least about 1.0%, and usually less than about 50% by weight, more usually less than about 10% by weight.

### Vasodilators

The subject compositions may also include vasodilating agents that function as active ingredients on their own, as well as facilitating the uptake of other active ingredients, thereby providing for a synergistic combination.

An exemplary vasodilator that finds us in the cosmetic compositions of the present invention is niacinamide (a vitamin B₃ compound), which aids in the penetration and uptake of active ingredients but does not cause the flushing and/or irritation of the skin associated with other vasodilators. This feature makes niacinamide useful in the subject cosmetic compositions because flushing and/or irritation of the skin would decrease the desirability and usefulness of the cosmetic compositions described herein. The niacinamide is usually present at a concentration of at least about 0.25% to 0.5%, more usually at least about 1 %, and not more than about 5%.

### Proteins

Thymosin-beta-4 (TB4) is a small polypeptide that inhibits the migration of macrophages, and stimulates the secretion of hypothalamic luteinizing hormone-releasing hormone. It has also been implicated in wound healing, (see Malinda et al. (1999) J. Invest. Dermatol. 113:364-368). The molecule is ubiquitous; it had been found in all tissues and cell lines analyzed, but is found in highest concentrations in spleen, thymus, lung, and peritoneal macrophages. The polypeptide sequence of TB4 may be found in U.S. Patent no. 4,297,276, Goldstein *et al.* The corresponding genetic sequence is described by Gondo et al. (1987) J. Immunol. 139 (11), 3840-3848, Genbank accession number M17733. Preferably human recombinant TB4 is used, which is commercially available, *e.g.* from Advanced ChemTech, Inc. (Louisville, KY), at a specific activity of 5 mg/1000 U. In the compositions of the present invention, the TB4 is used at a concentration of at least about 1 ng/ml, usually at least about 10 ng/ml, more usually at least about 100 ng/ml, and not more than about 10 µg/ml, more usually not more than about 1 µg, and may be used at a concentration of about 0.1 to 0.5 µg/ml.

Transforming growth factor beta 1 (TGFB) is a multifunctional peptide that controls proliferation, differentiation, and other functions in many cell types. Many cells synthesize TGFB and almost all of them have specific receptors for this peptide. The effect of TGFB on wound healing angiogenesis has been explored by Knighton et al. (1990) J. Trauma 30:S134-144. The polypeptide sequence of human TGFB and corresponding genetic sequence may be found in Genbank, accession number X02812 J05114, Derynck et al. (1985) Nature 316 (6030), 701-705. Recombinant human TGFB is commercially available, e.g. from R&D Systems (Minneapolis, MN), at a specific activity of 20 µg/1000 U. In the compositions of the present invention, when present TGFB1 is used at a concentration of at least about 5 pg/ml, usually at least about 50 pg/ml, more usually at least about 0.1 ng/ml, and not more than about 1 µg/ml, usually not more than about 100 ng/ml, more usually not more than about 10 ng/ml, and may be used at a concentration of from about 0.1 to 1 ng/ml.

For use in the present invention these proteins variants and active fragments of these proteins, as known in the art, may be used. The proteins may be produced from eukaryotic or prokaryotic cells by recombinant methods, isolated from cells in a native form, or may be synthesized *in vitro* as known in the art.

### Skin Soothing/Conditioning agents

The cosmetic compositions of the present invention may also contain agents that sooth, condition and/or heal the skin. One such agent is panthenol, a pro-vitamin moisturizing agent related to Vitamin E. Panthenol is easily incorporated into cosmetic formulations and readily penetrates the skin and hair. Panthenol derivatives (e.g., ethyl panthenol) also find use in the compositions of the invention as do agents such as aloe vera, pantothenic acid and its derivatives, allantoin, bisabolol, and dipotassium glycyrrhizinate. Many other skin conditioning/soothing agents can be included in the subject compositions, some of which are discussed below.

### Optional Skin Benefit Materials and Cosmetic Adjuncts

The compositions of the invention may optionally include other beneficial materials. These include steroidal hormones; progesterone; pregnanalone; coenzyme Q10; methylsolanomethane (MSM); copper peptide (copper extract); plankton extract (phytosome); transforming growth factor beta 1 (TGF-β1); glycolic acid; kojic acid; ascorbyl palmitate; all-trans-retinol; azaleic acid; salicylic acid; analgesics; non-steroidal anti-inflammatory drugs (NSAIDs); broparoestrol; *etc.* lf present, steroids will generally be present at a concentration of less than about 2% of the total by weight of the composition, while the other skin benefit materials may be present at higher levels, for example as much as 10 to 15%.

The compositions may further comprise sunscreens to lower skin's exposure to harmful UV rays. Sunscreens include those materials commonly employed to block ultraviolet light. Illustrative compounds are the derivatives of PABA, cinnamate and derivatives of salicylate (other than ferulyl salicylate). For example, octyl methoxycinnamate and 2-hydroxy-4-methoxy benzophenone (also known as oxybenzone) can be used. Octyl methoxycinnamate and 2-hydroxy-4-methoxy benzophenone are commercially available under the trademarks, Parsol MCX and Benzophenone-3, respectively. Dermascreen may also be used. Additionally, a composition may further include aloe vera or an extracted component or powder thereof, such as anthraquinone glycosides, resins, polysaccharides, sterols, gelonins, and chromones. The exact amount of sunscreen or aloe employed in the compositions can vary depending upon the degree of protection desired from the sun's UV radiation.

The cosmetic compositions of the present invention may also contain benzoyl peroxide, which may act synergistically with the various herbal active agents in certain cosmetic compositions of the invention to treat aging-related conditions and/or disorders. Benzoyl peroxide is a commonly used topical treatment for mild acne. Benzoyl peroxide has antiseptic properties, i.e., it reduces the number of skin surface bacteria and yeast; is an oxidizing agent, making it keratolytic and comedolytic; and has anti-inflammatory activity. The cosmetic compositions may contain benzoyl peroxide at concentrations contain at least about 1%, at least about 2.5%, at least about 5%, and usually not more than about 10% (weight/weight). Benzoyl peroxide is commercially available as a cream, gel, lotion, or wash under the following brand names: Benoxyl™, Benzac™, Brevoxyl™, Oxy™ and PanOxyl™.

### Cosmetically Acceptable Vehicle

The compositions of the invention comprise a cosmetically acceptable vehicle to act as a dilutant, dispersant or carrier for the herbal ingredients, so as to facilitate its distribution and uptake when the composition is applied to the skin. Vehicles other than or in addition to water can include liquid or solid emollients, solvents, humectants, thickeners, powders, and perfumes.

The cosmetically acceptable vehicle will usually form from 5% to 99.9%, preferably from 25% to 80% by weight of the composition, and can, in the absence of other cosmetic adjuncts, form the balance of the composition.

The compositions may be in the form of aqueous, aqueous/alcoholic or oily solutions; dispersions of the lotion or serum type; anhydrous or lipophilic gels; emulsions of liquid or semi-liquid consistency, which are obtained by dispersion of a fatty phase in an aqueous phase (O/W) or conversely (W/O); or suspensions or emulsions of smooth, semi-solid or solid consistency of the cream or gel type. These compositions are formulated according to the usual techniques as are well known to this art.

When the compositions of the invention are formulated as an emulsion, the proportion of the fatty phase may range from 5% to 80% by weight, and preferably from 5% to 50% by weight, relative to the total weight of the composition. Oils, emulsifiers and co-emulsifiers incorporated in the composition in emulsion form are selected from among those used conventionally in the cosmetic or dermatological field. The emulsifer and coemulsifier may be present in the composition at a proportion ranging from 0.3% to 30% by weight, and preferably from 0.5% to 20% by weight, relative to the total weight of the composition.

When the compositions of the invention are formulated as an oily solution or gel, the fatty phase may constitute more than about 50%, more than about 60%, more than about 70%, more than about 80%, more than about 90% of the total weight of the composition.

The compositions of the invention may be in the form of hair and body cleansing compositions. As such, these compositions may contain at least one wash-active surfactant in an aqueous base. The surfactants can be present, alone or in a mixture, and are contained in an amount of from 1 to about 50% by weight or from 1 to about 30% by weight. Nonionic surfactants, amphoteric surfactants, zwitterionic surfactants and anionic surfactants are generally suitable.

Suitable anionic surfactants include, e.g. alkaline or alkaline earth salts, alpha-olefin sulfonates, sulfosuccinates, disodium laureth-3 sulfosuccinate, disodium PEG-5 lauryl citrate sulfosuccinate, disodium ricinolamido MEA-sulfosuccinate or disodium laurylamido MEA-sulfosuccinate and alkyl ether carboxylates.

. Suitable nonionic surfactants include e.g., alkoxylated fatty alcohols, alkoxylated fatty acid esters, alkoxylated partial glycerides, saturated or unsaturated fatty acids, alkoxylated polyol esters, and alkylpolyglucosides, such as coconut glucosides, lauryl glycosides or decylglucosides. For example, ethoxylated lauryl alcohol, tetradecyl alcohol, cetyl alcohol, oleyl alcohol or stearyl alcohol, which are used alone or in mixtures with each other, as well as fatty alcohols of ethoxylated lanolin, are suitable as fatty alcohol ethoxylates. Furthermore the ethoxylated fatty acid sugar esters known as nonionic surfactants, especially ethoxylated sorbitan fatty acid ester, are suitable for use in the cosmetic preparations according to the invention. The suitable ethoxylated fatty acid sugar esters include those marketed under the trade names Tween™ and Arlacel™ by ICI surfactants and the alkyl-polyglycosides, which are marketed under the trade names Plantaren™ or Plantacare™ by Henkel or under the trade name Oramix™ by Seppic.

Suitable amphoteric surfactants include for example betaines, such as cocoamidopropylbetaine or lauryl betaine, sulfobetaines, such as cocoamidopropyl hydroxysultaine, glycinates, such as cocoamphoglycinate (lNCl-name: sodium cocoamphoacetate) and diglycinates and propionates, such as cocoampho-propionate.

The compositions of the invention may also contain additives and adjuvants which are conventional in the cosmetic, pharmaceutical or dermatological field, such as hydrophilic or lipophilic gelling agents, hydrophilic or lipophilic active agents, preservatives, antioxidants, solvents, fragrances, perfumes, fillers, bactericides, odor absorbers and dyestuffs or colorants. The amounts of these various additives and adjuvants are those conventionally used in the field, and, for example, range from 0.01% to 10% of the total weight of the composition. Depending on their nature, these additives and adjuvants may be introduced into the fatty phase or into the aqueous phase.

Exemplary oils which may be used according to this invention include mineral oils (liquid petrolatum), plant oils (liquid fraction of karite butter, sunflower oil), animal oils (perhydrosqualen(e), synthetic oils (purcellin oil), silicone oils (cyclomethicone) and fluoro oils (perfluoropolyethers). Fatty alcohols, fatty acids (stearic acid) and waxes (paraffin wax, carnauba wax and beeswax) may also be used as fats.

Emulsifiers which may be used include glyceryl stearate, polysorbate 60, PEG-6/PEG-32/glycol stearate mixture, etc. Solvents which may be used include the lower alcohols, in particular ethanol and isopropanol, and propylene glycol.

Hydrophilic gelling agents include carboxyvinyl polymers (carbomer), acrylic copolymers such as acrylate/alkylacrylate copolymers, polyacrylamides, polysaccharides, such as hydroxypropylcellulose, natural gums and clays, and, as lipophilic gelling agents, representative are the modified clays such as bentones, fatty acid metal salts such as aluminum stearates and hydrophobic silica, or ethylcellulose and polyethylene.

An oil or oily material may be present, together with an emollient to provide either a water-in- oil emulsion or an oil-in-water emulsion, depending largely on the average hydrophilic-lipophilic balance (HLB) of the emollient employed. Levels of such emollients may range from about 0.5% to about 50%, preferably between about 5% and 30% by weight of the total composition. Emollients may be classified under such general chemical categories as esters, fatty acids and alcohols, polyols and hydrocarbons.

Esters may be mono- or di-esters. Acceptable examples of fatty di-esters include dibutyl adipate, diethyl sebacate, diisopropyl dimerate, and dioctyl succinate. Acceptable branched chain fatty esters include 2-ethyl-hexyl myristate, isopropyl stearate and isostearyl palmitate. Acceptable tribasic acid esters include triisopropyl trilinoleate and trilauryl citrate. Acceptable straight chain fatty esters include lauryl palmitate, myristyl lactate, oleyl eurcate and stearyl oleate. Preferred esters include coco-caprylate/caprate (a blend of coco-caprylate and coco-caprate), propylene glycol myristyl ether acetate, diisopropyl adipate and cetyl octanoate.

Suitable fatty alcohols and acids include those compounds having from 10 to 20 carbon atoms. Especially preferred are such compounds such as cetyl, myristyl, palmitic and stearyl alcohols and acids.

Among the polyols which may serve as emollients are linear and branched chain alkyl polyhydroxyl compounds. For example, propylene glycol, sorbitol and glycerin are preferred. Also useful may be polymeric polyols such as polypropylene glycol and polyethylene glycol. Butylene and propylene glycol are also especially preferred as penetration enhancers.

Exemplary hydrocarbons which may serve as emollients are those having hydrocarbon chains anywhere from 12 to 30 carbon atoms. Specific examples include mineral oil, petroleum jelly, squalene and isoparaffins.

Another category of functional ingredients within the cosmetic compositions of the present invention for improving the appearance of the skin are thickeners. A thickener will usually be present in amounts anywhere from 0.1 to 20% by weight, preferably from about 0.5% to 10% by weight of the composition. Exemplary thickeners are cross-linked polyacrylate materials available under the trademark Carbopol. Gums may be employed such as xanthan, carrageenan, gelatin, karaya, pectin and locust beans gum. Under certain circumstances the thickening function may be accomplished by a material also serving as a silicone or emollient. For instance, silicone gums in excess of 10 centistokes and esters such as glycerol stearate have dual functionality.

Powders may be incorporated into the cosmetic composition of the invention. These powders include chalk, talc, kaolin, starch, smectite clays, chemically modified magnesium aluminum silicate, organically modified montmorillonite clay, hydrated aluminum silicate, fumed silica, aluminum starch octenyl succinate and mixtures thereof.

Other adjunct minor components may also be incorporated into the cosmetic compositions for improving the appearance of the skin. These ingredients may include coloring agents, opacifiers and perfumes. Amounts of these other adjunct minor components may range anywhere from 0.001 % up to 20% by weight of the composition.

### Product Use, Form, and Packaging

In use, a quantity of the composition, for example from 1 to 100 ml, is applied to a site of interest (e.g., skin, scalp, etc.) from a suitable container or applicator and, if necessary, it is then spread over and/or rubbed into the site using the hand or fingers or a suitable device. The composition may be specifically formulated for use as a treatment for a specific area, e.g. to approve the appearance of the hands, the face, the scalp, the feet, etc.

The cosmetic composition of the invention can be formulated in any form suitable for application to the site of interest, including a lotion, cream, gel, etc. The composition can be packaged in a suitable container to suit its viscosity and intended use by the consumer. For example, a lotion or cream can be packaged in a bottle, or a propellant-driven aerosol device or a container fitted with a pump suitable for finger operation. When the composition is a cream, it can simply be stored in a non-deformable bottle or squeeze container, such as a tube or a lidded jar. The invention accordingly also provides a closed container containing a cosmetically acceptable composition as herein defined.

The following examples are put forth so as to provide those of ordinary skill in the art with a complete disclosure and description of how to make and use the subject invention, and are not intended to limit the scope of what is regarded as the invention. Efforts have been made to insure accuracy with respect to the numbers used (e.g. amounts, temperature, concentrations, *etc*.) but some experimental errors and deviations should be allowed for. Unless otherwise indicated, parts are parts by weight, molecular weight is weight average molecular weight, temperature is in degrees centigrade, and pressure is at or near atmospheric.

### EXAMPLE 1

Example 1 illustrates topical compositions for improving the appearance of the skin according to the present invention. The compositions are illustrative of compositions of the invention comprising from 3 to 6% *Bacopa monnieri* extract, from 1.5 to 3% *Camellia sinensis* extract; from 1.5 to 3% *Curcuma longa* extract, from 4 to 8% *Silybum marianum* extract and from 3 to 6% *Withania somnifera* extract. The compositions can be processed in conventional manner. They are suitable for cosmetic use. In particular the compositions are suitable for application to a site of interest that has an aging-related condition or disorder that can occur in women in the transition phase of menopause. Application of the cosmetic compositions will combat these conditions thereby restoring a more youthful appearance. In addition, certain of these cosmetic compositions can be used to prevent the onset of the aging-related condition or disorder.
OIL-IN-WATER EMULSION I

| Ingredient | % w/w |
|---|---|
| Carbomer | 0.30 |
| Disodium EDTA | 0.10 |
| Glycerin | 3.00 |
| Polysorbate 20 | 2.50 |
| Butylene Glycol | 2.00 |
| Methylparaben | 0.30 |
| Triethanolamine 99% | 0.30 |
| Milk Thistle extract | 4.00% |
| Isopropyl Myristate | 5.00 |
| Octyl Palmitate | 3.00 |
| Cetyl Alcohol | 1.00 |
| Dimethicone 100 cst | 0.50 |
| Bacopa monniera extract | 3.00% |
| Beeswax | 0.30 |
| Turmeric extract | 1.50% |
| Propylparaben | 0.10 |
| Germall II | 0.10 |
| Ashwagandha powder | 3.00% |
| Fragrance | 0.10 |
| niacinamide | 1% |
| Green Tea | 1.50% |
| dl water to Total | 100.00 |

(optionally including 1 % benzoyl peroxide)
OIL-IN-WATER EMULSION II

| Ingredient | % w/w |
|---|---|
| Dl Water | |
| Carbomer | 0.30 |
| Disodium EDTA | 0.10 |
| Glycerin | 3.00 |
| Polysorbate 20 | 2.50 |
| Butylene Glycol | 2.00 |
| Methylparaben | 0.30 |
| Triethanolamine 99% | 0.30 |
| Isopropyl Myristate | 5.00 |
| Octyl Palmitate | 3.00 |
| Cetyl Alcohol | 1.00 |
| Dimethicone 100 cst | 0.50 |
| Beeswax | 0.30 |
| Propylparaben | 0.10 |
| Germall II | 0.10 |
| Fragrance | 0.10 |
| niacinamide | 1% |
| Milk Thistle extract | 8% |
| Bacopa monniera extract | 6% |
| Turmeric extract | 3% |
| Ashwagandha powder | 6% |
| Green Tea | 3% |
| dl water to Total | 100.00 |
| | |

(optionally including 1 % benzoyl peroxide)
OIL-IN-WATER EMULSION

| Ingredient | % w/w |
|---|---|
| Xanthan Gum | 0.20 |
| Disodium EDTA | 0.10 |
| Glycerin | 5.00 |
| Butylene Glycol | 2.00 |
| Methylparaben | 0.30 |
| Isopropyl Myristate | 5.00 |
| Octyl Palmitate | 3.00 |
| Cetyl Alcohol | 1.00 |
| Dimethicone 100 cst | 0.50 |
| Steareth-2 | 0.40 |
| Steareth-21 | 3.00 |
| Propylparaben | 0.10 |
| Germall II | 0.10 |
| Fragrance | 0.10 |
| niacinamide | 1% |
| Milk Thistle extract | 8% |
| Bacopa monniera extract | 6% |
| Turmeric extract | 3% |
| Ashwagandha powder | 6% |
| Green Tea | 3% |
| dl water to Total | 100.00 |

(optionally including 1% benzoyl peroxide)
WATER-IN-OIL EMULSION

| Ingredient | % w/w |
|---|---|
| Disodium EDTA | 0.10 |
| Glycerin | 3.00 |
| Propylene Glycol | 2.00 |
| Sodium Chloride | 0.70 |
| Methylparaben | 0.30 |
| Cyclomethicone | 14.00 |
| Isopropyl Myristate | 5.00 |
| Octyl Palmitate | 3.00 |
| Dimethicone Copolyol | 2.50 |
| Dimethicone 100 cst | 0.50 |
| Beeswax | 0.30 |
| Propylparaben | 0.10 |
| Germall ll | 0.10 |
| Fragrance | 0.10 |
| niacinamide | 1% |
| Milk Thistle extract | 8% |
| Bacopa monniera extract | 6% |
| Turmeric extract | 3% |
| Ashwagandha powder | 6% |
| Green Tea | 3% |
| dl water to Total | 100.00 |

(optionally including 1% benzoyl peroxide)
HYDRO-GEL

| ingredient | % w/w |
|---|---|
| Butylene Glycol | 5.00 |
| PPG-5-Ceteth 20 | 5.00 |
| Glycerin | 3.00 |
| Carbomer | 1.20 |
| Triethanolamine 99% | 1.20 |
| Methylparaben | 0.30 |
| Polysorbate 20 | 0.25 |
| Disodium EDTA | 0.10 |
| Germall II | 0.10 |
| niacinamide | 1% |
| Milk Thistle extract | 8% |
| Bacopa monniera extract | 6% |
| Turmeric extract | 3% |
| Ashwagandha powder | 6% |
| Green Tea | 3% |
| dl water to Total | 100.00 |

(optionally including 1% benzoyl peroxide)
ANHYDROUS SERUM

| ingredient | % w/w |
|---|---|
| Cyclomethicone | 71.40 |
| Isopropyl Myristate | 5.00 |
| Octyl Palmitate | 3.00 |
| Polyglycerol-6 Dioleate | 5.00 |
| Butylene Glycol | 4.00 |
| Dimethicone, 100 cst | 5.00 |
| Beeswax | 0.30 |
| Propylparaben | 0.20 |
| Fragrance | 0.10 |
| niacinamide | 1% |
| Milk Thistle extract | 8% |
| Bacopa monniera extract | 6% |
| Turmeric extract | 3% |
| Ashwagandha powder | 6% |
| Green Tea | 3% |
| Cyclomethicone to Total | 100.00 |

(optionally including 1% benzoyl peroxide)
HYDRO-ALCOHOLIC GEL

| ingredient | % w/w |
|---|---|
| Alcohol SDA40B | 30.00 |
| Butylene Glycol | 5.00 |
| PPG-5-Ceteth 20 | 5.00 |
| Glycerin | 3.00 |
| Carbomer | 1.20 |
| Triethanolamine 99% | 1.20 |
| 4-chromanone | 1.00 |
| Methylparaben | 0.30 |
| Polysorbate 20 | 0.25 |
| Disodium EDTA | 0.10 |
| Germall II | 0.10 |
| niacinamide | 1% |
| Milk Thistle extract | 8% |
| Bacopa monniera extract | 6% |
| Turmeric extract | 3% |
| Ashwagandha powder | 6% |
| Green Tea | 3% |
| dl Water to Total | 100.00 |

(optionally including 1% benzoyl peroxide)

All publications and patent applications cited in this specification are herein incorporated by reference as if each individual publication or patent application were specifically and individually indicated to be incorporated by reference.

Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended claims.

## Claims

1. A cosmetic composition for topical treatment of skin, comprising at least one active agent as an extract of *Bacopa monnieri, Camellia sinensis, Curcuma longa, Silybum marianum, Withania somnifera,* and a cosmetically acceptable vehicle.

2. The cosmetic composition according to claim 1, wherein said *Bacopa monnieri* extract is present at a concentration of about 2 weight percent to about 20 weight percent of the composition.

3. The cosmetic composition according to claim 2, wherein said *Bacopa monnieri* extract comprises bacosides.

4. The cosmetic composition according to any one of the preceding claims, wherein said *Camellia sinensis* extract is present at a concentration of about 2 weight percent to about 20 weight percent of the composition.

5. The cosmetic composition according to any one of the preceding claims, wherein said *Camellia sinensis* extract comprises silymarin.

6. The cosmetic composition according to any one of the preceding claims, wherein said *Curcuma longa* extract is present at a concentration of about 2 weight percent to about 20 weight percent of the composition.

7. The cosmetic composition according to claim 6, wherein said *Curcuma longa* extract comprises curcumin.

8. The cosmetic composition according to any one of the preceding claims, wherein said *Silybum marianum* extract is present at a concentration of about 2 weight percent to about 20 weight percent of the composition.

9. The cosmetic composition according to claim 8, wherein said *Silybum marianum* extract comprises polyphenols.

10. The cosmetic composition according to any one of the preceding claims, wherein said *Withania somnifera* extract is present at a concentration of about 2 weight percent to about 20 weight percent of the composition.

11. The cosmetic composition according to any one of the preceding claims, comprising:
from 1 to 20% by weight *Bacopa monnieri* extract;
from 1 to 20% by weight *Camellia sinensis* extract;
from 1 to 20% by weight *Curcuma longa* extract;
from 1 to 20% by weight *Silybum marianum* extract;
from 1 to 20% by weight *Withania somnifera* extract; and
a cosmetically acceptable vehicle.

12. The cosmetic composition according to claim 11, further comprising an acylated peptide that is present from about 1 to 10% by weight of said composition.

13. The cosmetic composition according to claim 11 or 12, further comprising a non-flushing vasodilating agent.

14. The cosmetic composition of claim 13, wherein said vasodilating agent is niacinamide and is present at 0.5 to 5 % by weight of said composition.

15. The composition according any one of the preceding claims, wherein said cosmetically acceptable vehicle is an oil in water, or water in oil emulsion.

16. The composition according to any one of the preceding claims, wherein said composition further comprises a sunblock.

17. A method for improving the appearance of aged, photoaged, dry, inflamed, lined or wrinkled skin, the method comprising applying topically a cosmetic composition of any one of claims 1 to 16.

18. The cosmetic composition of any one of claims 1 to 16 for use in a method for topically treating a condition of the skin.
